# EUROPEAN PATENT SPECIFICATION

(11) **EP 0 690 042 B1**
(45) Date of publication and mention of the grant of the patent: **07.10.1998**
(21) Application number: 95113235.6
(22) Date of filing: 18.02.1992
(51) Int. Cl.: C07C 209/84

(54) **Process for producing aliphatic amines**
Verfahren zur Herstellung von aliphatischen Aminen
Procédé de préparation d'amines aliphatiques

(30) Priority: 22.02.1991 JP 28420/91
(43) Date of publication of application: 03.01.1996
(62) Divisional of application: 92102670.4
(73) Proprietor: Kao Corporation, Chuo-Ku Tokyo 103 (JP)
(72) Inventor: Abe, Hiroshi, Albany, CA 94706 (US); Taniguchi, Hideki, Naga-gun, Wakayama (JP); Tachizawa, Osamu, Wakayama-shi, Wakayama (JP); Sotoya, Kohshiro, Naga-gun, Wakayama (JP)
(74) Representative: Hansen, Bernd, Dr. Dipl.-Chem.

(56) References cited:
- EP-A- 0 312 253
- DE-A- 1 768 743
- DE-B- 1 162 848
- GB-A- 717 680

## Description

The present invention relates to a process for producing an aliphatic tertiary amine.

Aliphatic tertiary amines prepared from tallow, coconut oil, palm oil and the like are important intermediates for domestic and industrial products.

Aliphatic tertiary amines are used in a wide field includes a softener for clothes, an antistatic agent, a gasoline additive, a shampoo, a conditioner, a microbicide and a detergent.

With respect to the preparation of an aliphatic amine, there has been known a process for producing such an amine from a fat and/or an oil through a fatty acid and a nitrile and a process for producing an amine from a fat and/or an oil through a higher alcohol (See "Kazuhiko Okabe and Hirosi Abe, Yukagaku", Vol.37, No.7, p.487, 1988). However, aliphatic amines prepared by these conventional processes have problems such that they are colored in the conversion thereof into derivatives and also they become turbid when stored for a long time to give products which are poor in appearance.

GB-A-717 680 discloses the purification of alkylated phenylene diamines by treating these compounds with alkaline or basic substances. An aqueous or alcoholic solution of the alkali or base may be added to the crude diamine or substantially pure diamine distilled off from the mixture by fractional distillation under reduced pressure conditions. According to the Examples, phenylene diamine was obtained by reacting p-nitroaniline with methyl ethyl ketone.

DE-A-17 68 743 discloses the purification of primary, secondary and tertiary amines by heating these amines in the presence of alkalis and by subsequently performing a distillation. This document describes that primary, secondary and tertiary amines are prepared from the corresponding nitriles by a catalytic hydrogenation in the presence of ammonia or a primary or secondary amine.

EP-A-0 312 253 reveals a process for preparing N-substituted amines by reacting an alcohol or aldehyde with ammonia, an amine in the presence of a specific catalyst. The amine may be a primary amine or a secondary amine such as dimethyl amine. The catalyst is necessary for removing water which is formed during the reaction. After completion of the reaction the reaction mixture itself is subjected to distillation or filtration to separate the reactant from the catalyst. The N-substituted amine obtained by the filtration can be distilled into a highly pure form.

Under these circumstances, the object of the present invention is to provide a process for producing high-quality aliphatic tertiary amines which can be converted into useful derivatives without suffering from coloration or turbidity even after long-term storage.

The present invention provides a process for producing an aliphatic tertiary amine comprising a synthesis step of an aliphatic amine by reacting aliphatic primary alcohol with dimethylamine to give an aliphatic tertiary amine, (step I) and at least one purifying step (step II) by contacting the aliphatic amine with an aqueous solution of an inorganic alkali, separating and recovering the aliphatic amine from the aqueous solution of an inorganic alkali and/or contacting the aliphatic amine with water, separating and recovering the aliphatic amine from the water.

The aliphatic primary alcohol to be used is preferably a saturated or unsaturated aliphatic primary alcohol having 8 to 22 carbon atoms and the reaction of this step is represented by the following reaction formula: 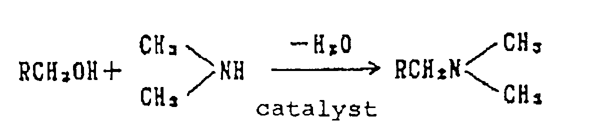 wherein R is a saturated or unsaturated aliphatic group having 7 to 21 carbon atoms.

In this step, an aliphatic primary alcohol and dimethylamine are used at a molar ratio of about 1.0 : 1.0 to 5.0 and a catalyst comprising Cu or Ni as the main component is used. Examples of the catalyst include Cu-Ni, Cu-Zn and Cu-Co and catalysts prepared by adding at least one element selected from among Pd, Pt, Ru and Rh among Group VIII platinum metals to these catalyst. The amount of the catalyst to be used is 0.1 to 5% by weight based on the aliphatic primary alcohol. The reaction temperature is generally 150 to 230°C and the pressure is from atmospheric pressure to 100 atm (gauge pressure).
wherein R is a saturated or unsaturated aliphatic group having 7 to 21 carbon atoms.

The catalyst and reaction conditions to be employed in this step may be each selected from among those usable in step (e).

The aliphatic tertiary amines (reaction product aliphatic amines, including impurities) prepared by the above reaction include those having 8 to 66 carbon atoms in total and particular examples thereof include N,N-dimethyldodecylamine N,N-dimethylhexadecylamine and N,N-dimethyloctadecylamine.

The reaction product aliphatic amine prepared by step I is then purified by at least one purification step.

The amount of water or aqueous solution of an inorganic alkali to be used in this step is 0.01 to 20% by weight based on the weight of the reaction product aliphatic amine. The contact of the reaction product aliphatic amine with water or the aqueous solution of an inorganic alkali is conducted while stirring at room temperature to 150°C for 0.1 to 10 hours. The inorganic alkali is preferably at least one compound selected from alkali metal hydroxides, alkali metal carbonates and alkali metal hydrogencarbonates and specific examples thereof include sodium hydroxide, potassium hydroxide sodium carbonate, potassium carbonate, sodium hydrogencarbonate and potassium hydrogencarbonate which may be used in various forms such as powder, aqueous solution and flake. The amount of the inorganic alkali to be used is 0,001 to 5% by weight based on the weight of the reaction product aliphatic amine. The concentration of the aqueous solution is preferably 0.1 to 50% by weight.

The process of the present invention provides a high-quality aliphatic amine which can be converted into a derivative without suffering from coloration and does not become turbid even after long-term storage.

### Reference Example 1 and Comparative Example 1

600 g of dodecyl alcohol (a product of Kao Corporation; Kalcohl 20) and 3 g (corresponding to 0.5% by weight based on the alcohol used as the raw material) of a copper-nickel catalyst were fed into a 1-ℓ four-necked flask. The system was purged with nitrogen while stirring and the temperature rise of the system was initiated. When the temperature of the system reached 100°C, hydrogen gas was blown into the system at a flow rate of 40 ℓ/hr with a flowmeter and the temperature of the system was raised to a reaction temperature, i.e., 200°C. At this temperature, the introduction of dimethylamine gas was initiated and a reaction was conducted for 5 hours. After the completion of the reaction, the reaction mixture was filtered to remove the catalyst, thereby providing crude N,N-dimethyldodecylamine. The crude N,N-dimethyldodecylamine was put in a 1-ℓ four-necked flask, followed by the addition of 3 g of activated carbon and 3 g of Kyoward 600 S (a product of Kyowa Chemical Industry Co., Ltd., main components: silica 64.9%, magnesia 13.5%, both listed values). The contents were stirred at 90°C in a nitrogen atmosphere for about 2 hours and filtered to remove the adsorbent. The filtrate was purified by distillation to give N,N-dimethyldodecylamine. The distillation was conducted at 5 Torr.

The amine prepared above (Reference Example 1) and the amine (Comparative Example 1) prepared by the same procedure as that described above except that no treatment with activated carbon and Kyoward 600S was conducted prior to the distillation, were examined for quality just after preparation and after storage in air at 60°C for one month by the following method and the results are given in Table 1.

### 〈Test method of quality〉

A solution prepared by mixing 20 g of an amine sample with 20 g of ethanol and 10 g of 35% hydrochloric acid is kept at 60°C for 15 minutes to determine the color change of the solution with a Lovibond colorimeter (using a 5 1/4 inch cell).

**Table 1**

| | Lovibond Red | |
|---|---|---|
| | Reference Example 1 | Comparative Example 1 |
| treatment with activated carbon and Kyoward | made | not made |
| just after preparation | 0.1 or below | 0.1 or below |
| after the storage for one month | 0.1 or below | 22.7 |

### Examples 1 and 2 and Comparative Example 2

Crude N,N-dimethylstearylamine was prepared in a similar manner to that of Reference Example 1 except that stearyl alcohol (a product of Kao Corporation, Kalcohl 80) was used instead of the dodecyl alcohol. The crude N,N-dimethylstearylamine was treated by various methods specified in Table 2 and purified by distillation. The obtained amines were stored in the air at 30°C for one month to examine the appearance (whether they were turbid or clear).

The results are given in Table 2.

**Table 2**

| | Method for treating crude amine | Appearance after one month |
|---|---|---|
| Ex. 1 | 48% aqueous solution of NaOH, activated carbon and Kyoward 600S were added to crude amine (each in an amount 0.5% by weight based on the crude amine) and the obtained mixture was stirred at 90°C for 2 hours in a nitrogen atmosphere and filtered to remove the adsorbents. The obtained amine was purified by distillation and stored for one month. | clear |
| Ex. 2 | activated carbon and Kyoward 600S were added to crude amine (each in an amount of 0.5% by weight based on the crude amine) and the obtained mixture was stirred at 90°C for 2 hours in a nitrogen atmosphere and filtered to remove the adsorbents. 1% (based on the amine) of water was added to the filtrate and the obtained mixture was maintained at 50°C for about one hour, distilled and stored for one month. | clear |
| | | |
| Comp. Ex. 2 | no treatment was conducted to crude amine except for distillation and the obtained distillate was stored for one month. | turbid |

It can be understood from the above results that an amine which does not become turbid even after storage for one month can be obtained by treating a crude amine in accordance with the claimed process.

## Claims

1. A process for producing an aliphatic tertiary amine comprising a synthesis step of an aliphatic amine by reacting aliphatic primary alcohol with dimethylamine to give an aliphatic tertiary amine, (step I) and at least one purifying step (step II) by contacting the aliphatic amine with an aqueous solution of an inorganic alkali, separating and recovering the aliphatic amine from the aqueous solution of an inorganic alkali and/or contacting the aliphatic amine with water, separating and recovering the aliphatic amine from the water.

2. The process for producing a tertiary amine accoring to claim 1, wherein the inorganic alkali used is at least one compound selected from the group consisting of an alkali metal hydroxide, an alkali metal carbonate and an alkali metal hydrogen carbonate.

3. The process for producing a tertiary amine according to claim 1 or 2, wherein step II is conducted in an inert atmosphere.

4. The process for producing an aliphatic tertiary amine according to any one of the claims 1 to 3 wherein the aliphatic amine has a saturated or unsaturated aliphatic group having 8 to 22 carbon atoms.

## Patentansprüche

1. Verfahren zur Herstellung eines aliphatischen, tertiären Amins, umfassend einen Schritt der Synthese eines aliphatischen Amins durch Reaktion eines aliphatischen primären Alkohols mit Dimethylamin, unter Erhalt eines aliphatischen tertiären Amins (Schritt I) und zumindest einen Reinigungsschritt (Schritt II) durch Kontaktieren des aliphatischen Amins mit einer wäßrigen Lösung aus einem anorganischen Alkali, Trennen und Wiedergewinnen des aliphatischen Amins von der wäßrigen Lösung eines anorganischen Alkali und/oder Kontaktieren des aliphatischen Amins mit Wasser, Trennen und Wiedergewinnen des aliphatischen Amins von dem Wasser.

2. Verfahren zur Herstellung eines tertiären Amins nach Anspruch 1, worin das verwendete anorganische Alkali zumindest eine Verbindung ist bestehend aus der Gruppe, ausgewählt aus einem Alkalimetallhydroxid, einem Alkalimetallcarbonat und einem Alkalimetallhydrogencarbonat.

3. Verfahren zur Erzeugung eines tertiären Amins nach Anspruch 1 oder 2, worin der Schritt II in einer inerten Atmosphäre durchgeführt wird.

4. Verfahren zur Herstellung eines aliphatische, tertiären Amins nach einem der Ansprüche 1 bis 3, worin das aliphatische Amin eine gesättigte oder ungesättigte, aliphatische Gruppe mit 8 bis 22 Kohlenstoffatomen aufweist.

## Revendications

1. Procédé de production d'une amine aliphatique tertiaire comprenant une étape de synthèse d'une amine aliphatique consistant à faire réagir un alcool aliphatique primaire avec de la diméthylamine pour obtenir une amine aliphatique tertiaire (étape I) et au moins une étape de purification (étape II) consistant à mettre en contact l'amine aliphatique avec une solution aqueuse d'une base inorganique, à séparer et à récupérer l'amine aliphatique à partir de la solution aqueuse d'une base inorganique et/ou à mettre en contact l'amine aliphatique avec de l'eau, à séparer et à récupérer l'amine aliphatique à partir de l'eau.

2. Procédé de production d'une amine tertiaire selon la revendication 1, dans lequel la base inorganique utilisée est au moins un composé choisi dans le groupe constitué par un hydroxyde de métal alcalin, un carbonate de métal alcalin et un carbonate acide de métal alcalin.

3. Procédé de production d'une amine tertiaire selon la revendication 1 ou 2, dans lequel on effectue l'étape II dans une atmosphère inerte.

4. Procédé de production d'une amine aliphatique tertiaire selon l'une quelconque des revendications 1 à 3, dans lequel l'amine aliphatique comporte un groupe aliphatique saturé ou insaturé ayant 8 à 22 atomes de carbone.
